# EUROPEAN PATENT APPLICATION

(11) **EP 3 355 047 A1**
(43) Date of publication of application: **01.08.2018**
(21) Application number: 17789060.5
(22) Date of filing: 28.02.2017
(51) Int. Cl.: G01N 21/51, G01N 21/03, G01N 21/59, G01N 33/28, G01N 33/18

(54) **FLUID STATE DETECTION SYSTEM**

(30) Priority: 27.04.2016 JP 2016089098
(71) Applicant: KYB Corporation, Tokyo 105-6111 (JP)
(72) Inventor: YOSHIDA, Takahiro, Tokyo 105-6111 (JP); KAMEDA, Yukinori, Tokyo 105-6111 (JP)
(74) Representative: Horn Kleimann Waitzhofer Patentanwälte PartG mbB
(86) International application number: PCT/JP2017/007847
(87) International publication number: WO 2017/187771

(57) **Abstract**

A fluid state detection system that enables the state of a fluid to be readily detected is provided. A fluid state detection system (1) for detecting the state of a fluid in a container (10) includes a measurement window (11) formed in a wall of the container (10), and a fluid state detection apparatus (20) for detecting the state of the fluid. The fluid state detection apparatus (20) includes a transmission unit (21), a receiving unit (22), and a detection unit (23). The transmission unit (21) emits electromagnetic waves via the measurement window (11) toward the fluid. The receiving unit (22) receives reflected waves, transmitted waves, or re-radiated scattered waves of the electromagnetic waves emitted from the transmission unit (21) via the measurement window (11) toward the fluid. The detection unit (23) detects the state of the fluid based on the electromagnetic waves emitted from the transmission unit (21) and the electromagnetic waves received by the receiving unit (22).

## Description

### Technical Field

The present invention relates to a fluid state detection system.

### Background Art

Techniques for detecting the state of a fluid have been proposed (e.g. see Patent Document 1).

Patent Document 1 describes an oil degradation detection apparatus for determining whether oil has been degraded, the oil being circulated and supplied to rotary and sliding portions in order to smoothly operate these parts while preventing abrasion thereof. This oil degradation detection apparatus includes two plates, which are installed parallel to each other in an oil passage. The apparatus obtains the permittivity and conductivity of the oil by applying an AC voltage across these two plates, and determines whether the oil has been degraded based on the permittivity and conductivity.

### Citation List

### Patent Literature

### [Patent Document 1]

Japanese Patent Application Laid Open No. 2009-2693

### Summary of Invention

### Technical Problem

In the oil degradation detection apparatus described in Patent Document 1, it is necessary to install two plates in the oil passage and provide structures for applying an AC voltage across these two plates. For this reason, major structural additions and modifications are required for the equipment in which the aforementioned rotary and sliding portions are provided. There has been a demand for a simpler way of detecting the state of a fluid.

The present invention has been made in view of the foregoing problem, and provides a fluid state detection system that enables the state of a fluid to be readily detected.

### Solution to problem

One or more embodiments of the present invention relate to a fluid state detection system for detecting a state of a fluid in a container, including: a measurement window formed in a wall of the container; and a fluid state detection apparatus for detecting the state of the fluid, wherein the fluid state detection apparatus comprises: a transmission unit for emitting electromagnetic waves via the measurement window toward the fluid; a receiving unit for receiving reflected waves, transmitted waves, or re-radiated scattered waves of the electromagnetic waves emitted from the transmission unit via the measurement window toward the fluid; and a detection unit for detecting the state of the fluid based on the electromagnetic waves emitted from the transmission unit and the electromagnetic waves received by the receiving unit.

### Brief Description of the Drawings

FIG. 1 is a schematic diagram showing a configuration of a fluid state detection system according to a first embodiment of the present invention.
FIG. 2 is a schematic diagram showing a configuration of a fluid state detection system according to a second embodiment of the present invention.

### Description of Embodiments

Hereinafter, embodiments of the present invention will be described with reference to the drawings. Note that the constituent elements in the following embodiments may be replaced with existing constituent elements as appropriate. Also, different variations thereof including combinations with other existing constituent elements are possible. Accordingly, the following embodiments are not intended to limit the content of the invention described in the claims.

### First Embodiment

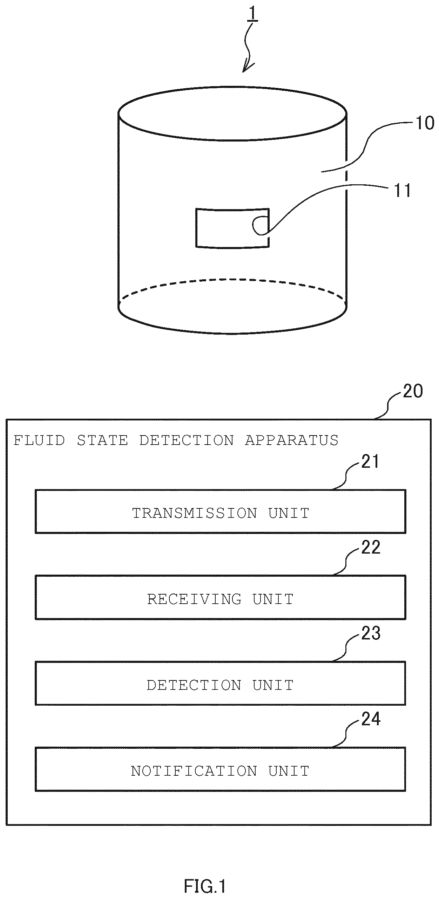

FIG. 1 is a schematic diagram showing a configuration of a fluid state detection system 1 according to the first embodiment of the present invention. The fluid state detection system 1 includes a container 10 in which lubricating oil is present, and a fluid state detection apparatus 20 for detecting the state of the lubricating oil in the container 10.

The container 10 is a pipe through which the lubricating oil flows, a region near a driving portion, such as a rotary portion or a sliding portion, a reservoir portion for storing the lubricating oil, or the like. A measurement window 11 is provided in a side wall of this container 10. The measurement window 11 is made of a material through which electromagnetic waves are transmitted, such as glass or resin.

The fluid state detection apparatus 20 includes a transmission unit 21, a receiving unit 22, a detection unit 23, and a notification unit 24. The transmission unit 21 emits electromagnetic waves via the measurement window 11 toward the lubricating oil in the container 10.

The receiving unit 22 receives reflected waves, transmitted waves, or re-radiated scattered waves of the electromagnetic waves emitted from the transmission unit 21 via the measurement window 11 toward the lubricating oil in the container 10. In the case of receiving the reflected waves or scattered waves, the receiving unit 22 receives the electromagnetic waves that exit from the inside of the container 10 via the measurement window 11. In the case of receiving the transmitted waves, a measurement window (hereinafter, "opposing measurement window") that is similar to the measurement window 11 is formed at a position on the side wall of the container 10, the position opposing the position at which the measurement window 11 is provided. Thus, the receiving unit 22 can receive, of the electromagnetic waves that are incident from the measurement window 11, the electromagnetic waves that are transmitted through the lubricating oil in the container 10 and exit from the opposing measurement window, and receive the transmitted waves.

The relationship between the intensity and polarization of the electromagnetic waves emitted from the transmission unit 21 and the intensity and polarization of the electromagnetic waves received by the receiving unit 22 varies depending on the amount of foreign substances contained in the lubricating oil in the container 10, such as metal, varnish, sludge, and water content, the degree of oxidation of the lubricating oil, and so on. The detection unit 23 detects the state of the lubricating oil in the container 10 based on the intensity and polarization of the electromagnetic waves emitted from the transmission unit 21, and the intensity and polarization of the electromagnetic waves received by the receiving unit 22.

The notification unit 24 gives a notification of the detection result from the detection unit 23. If the notification unit 24 is configured as a display unit for displaying characters or images, the notification unit 24 displays the detection result from the detection unit 23. If the notification unit 24 is configured as an audio unit for outputting sound, the notification unit 24 outputs a voice signal for announcing the detection result from the detection unit 23, or outputs a sound if the detection result is a predetermined detection result.

The aforementioned transmission unit 21, receiving unit 22, detection unit 23, and notification unit 24 are realized using a CPU, a memory (RAM), a hard disk, or the like.

The hard disk stores an operating system, a program for executing a series of processes for detecting the state of the lubricating oil, and the like. Note that the hard disk need only be a non-temporary recording medium, and may also be, for example, a nonvolatile memory such as an EPROM or a flash memory, a CD-ROM, or the like.

The CPU uses the memory to read out data and programs stored in the hard disk as appropriate, and computes and executes the same as appropriate.

As described above, the fluid state detection system 1 includes the container 10 that is provided with the measurement window 11, and the fluid state detection apparatus 20. Using the fluid state detection apparatus 20, electromagnetic waves are emitted toward the lubricating oil in the container 10 via the measurement window 11, reflected waves, transmitted waves, or re-radiated scattered waves of the electromagnetic waves are received, and the state of the lubricating oil in the container 10 is detected based on the emitted electromagnetic waves and the received electromagnetic waves. With this configuration, the state of the lubricating oil can be detected without major structural additions or modifications of the equipment. Accordingly, the state of the lubricating oil can be readily detected.

The liquid surface of the lubricating oil in the container 10 is not always even, and may be agitated, for example. For this reason, if electromagnetic waves are emitted toward the lubricating oil from vertically above the container 10, an uneven liquid surface of the lubricating oil may affect the electromagnetic waves received by the receiving unit 22, and there is a concern that the state of the lubricating oil cannot be detected appropriately. In addition, the foreign substances contained in the lubricating oil may precipitate. For this reason, if electromagnetic waves are emitted toward the lubricating oil from vertically below the container, the precipitated foreign substances may affect the electromagnetic waves received by the receiving unit 22, and there is a concern that the state of the lubricating oil cannot be detected appropriately in this case. In the fluid state detection system 1, the measurement window 11 is therefore provided in the side wall of the container 10. With this configuration, even if the liquid surface of the lubricating oil is not even, or even if foreign substances contained in the lubricating oil have precipitated, the state of the lubricating oil can be appropriately detected without being affected thereby.

Also, the fluid state detection system 1 includes the notification unit 24 for giving a notification of the detection result from the detection unit 23. Accordingly, a notification of the result of detecting the state of the lubricating oil in the container 10 can be given.

Note that, although the fluid state detection system 1 detects the state of the lubricating oil in this embodiment, the present invention is not limited thereto, and the state of any kind of fluid can be detected. For example, the fluid state detection system 1 can also detect the degree of contamination of water, and can also detect the quality of a chemical agent or a beverage.

In a pipe through which the lubricating oil flows, or in a region near a driving portion, such as a rotary portion or a sliding portion, the lubricating oil is flowing. Thus, bubbles may be generated in the lubricating oil, or the amount of the lubricating oil may vary. If bubbles are present in the lubricating oil, the bubbles may affect the electromagnetic waves received by the receiving unit 22, and there is a concern that the state of the lubricating oil cannot be detected appropriately. If the amount of the lubricating oil varies, the liquid surface of the lubricating oil may drop. Then, there is a concern that the electromagnetic waves emitted from the transmission unit 21 are not appropriately applied to the lubricating oil, and the state of the lubricating oil cannot be detected appropriately. In this embodiment, the container 10 may also be a reservoir portion for storing the lubricating oil, rather than the aforementioned pipe or the aforementioned region near the driving portion. The lubricating oil flows relatively gently in the reservoir portion, compared with the lubricating oil in the aforementioned pipe or the aforementioned region near the driving portion. Accordingly, bubbles are less likely to be generated in the lubricating oil, and the amount of the lubricating oil is less likely to vary. Thus, the state of the lubricating oil can be detected appropriately.

In this embodiment, the fluid state detection apparatus 20 may be a stationary apparatus, but it may also be a portable apparatus. If the fluid state detection apparatus 20 is a stationary apparatus, the position and angle of the fluid state detection apparatus 20 relative to the measurement window 11 can be fixed. With this configuration, every time the electromagnetic waves are emitted from the transmission unit 21, the electromagnetic waves can be applied to the lubricating oil in the container 10 from the same position at the same angle. Accordingly, the state of the lubricating oil can be detected appropriately.

On the other hand, if the fluid state detection apparatus 20 is a portable apparatus, the fluid state detection apparatus 20 can be carried, which is more convenient. Moreover, in this case, a laser pointer may also be provided in the fluid state detection apparatus 20 so that the electromagnetic waves emitted from the transmission unit 21 can be appropriately applied to the lubricating oil in the container 10 via the measurement window 11. In addition, to make the position and the angle of the fluid state detection apparatus 20 relative to the measurement window 11 fixed every time the electromagnetic waves are emitted from the transmission unit 21, a fixing portion for fixing the fluid state detection apparatus 20 may also be provided at a predetermined position, with the measurement window 11 serving as a reference.

### Second Embodiment

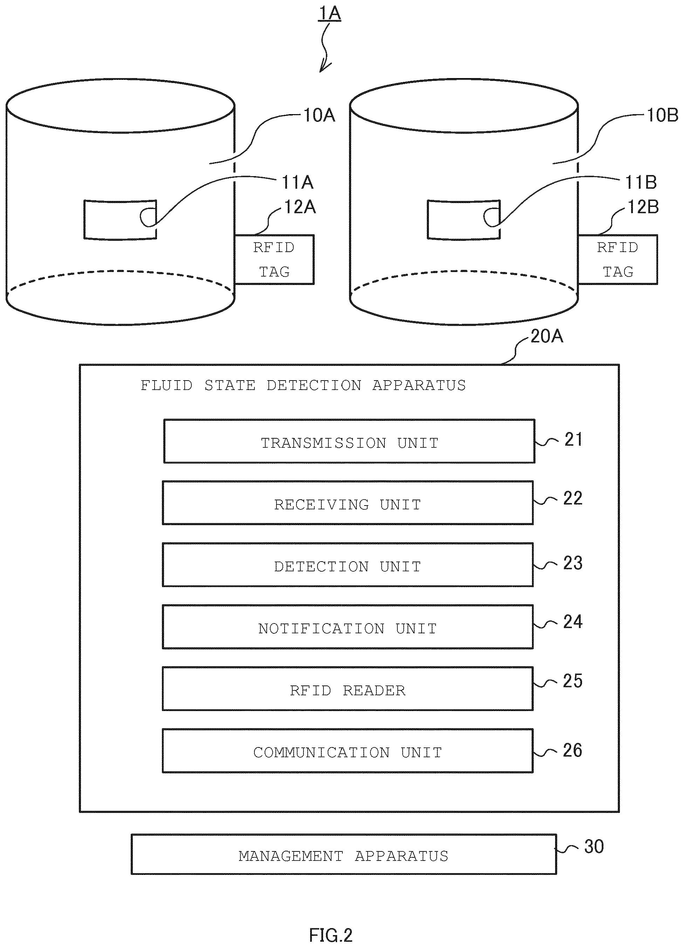

FIG. 2 is a schematic diagram showing a configuration of a fluid state detection system 1A according to the second embodiment of the present invention. The fluid state detection system 1A differs from the fluid state detection system 1 according to the first embodiment of the present invention shown in FIG. 1 in the points of including containers 10A and 10B in place of the container 10, including a fluid state detection apparatus 20A in place of the fluid state detection apparatus 20, and including a management apparatus 30. The other portions are the same. Accordingly, the same portions as those in the first embodiment are assigned the same signs, and descriptions thereof are omitted.

Similarly to the container 10, the containers 10A and 10B are pipes through which the lubricating oil flows, a region near a driving portion, such as a rotary portion or a sliding portion, reservoir portions for storing the lubricating oil, or the like. A measurement window 11A is provided in a side wall of the container 10A, and a measurement window 11B is provided in a side wall of the container 10B. Similarly to the measurement window 11, the measurement windows 11A and 11B are made of a material through which electromagnetic waves are transmitted. An RFID tag 12A, in which information is written that enables the container 10A to be uniquely identified, is attached to the container 10A. An RFID tag 12B, in which information is written that enables the container 10B to be uniquely identified, is attached to the container 10B.

The fluid state detection apparatus 20A includes an RFID reader 25 and a communication unit 26, in addition to the transmission unit 21, the receiving unit 22, the detection unit 23, and the notification unit 24. The RFID tags 12A and 12B and the RFID reader 25 function as an identification unit for identifying the containers 10A and 10B.

The fluid state detection apparatus 20A is a portable apparatus. Accordingly, the fluid state detection apparatus 20A can detect the state of the lubricating oil in the container 10A by applying the electromagnetic waves emitted from the transmission unit 21 via the measurement window 11A toward the lubricating oil in the container 10A, and can detect the state of the lubricating oil in the container 10B by applying the electromagnetic waves emitted from the transmission unit 21 toward the lubricating oil in the container 10B via the measurement window 11B.

The RFID reader 25 reads, as appropriate, the information written in the RFID tag 12A and the information written in the RFID tag 12B. When the electromagnetic waves are emitted toward the lubricating oil in the container 10A, the RFID reader 25 reads the information that enables the container 10A to be uniquely identified, from the RFID tag 12A. When the electromagnetic waves are emitted toward the lubricating oil in the container 10B, the RFID reader 25 reads the information that enables the container 10B to be uniquely identified, from the RFID tag 12B.

The communication unit 26 transmits, to the management apparatus 30, the state of the lubricating oil detected by the detection unit 23 and the information read by the RFID reader 25 in association with each other. Thus, the management apparatus 30 can identify whether the state of the lubricating oil detected by the detection unit 23 is the state of the lubricating oil in the container 10A or the container 10B. The management apparatus 30 stores the information transmitted from the communication unit 26.

As described above, in the fluid state detection system 1A, the measurement windows 11A and 11B are provided in side walls of the containers 10A and 10B, respectively, and the transmission unit 21, the receiving unit 22, the detection unit 23, and the notification unit 24 are provided in the fluid state detection apparatus 20A, similarly to the fluid state detection system 1 according to the first embodiment of the present invention. Accordingly, the fluid state detection system 1A can achieve the same effects as those of the fluid state detection system 1 according to the first embodiment of the present invention.

In addition, in the fluid state detection system 1A, the RFID tag 12A in which the information that enables the container 10A to be uniquely identified is written is attached to the container 10A, and the RFID tag 12B in which the information that enables the container 10B to be uniquely identified is written is attached to the container 10B. Also, the RFID reader 25 is provided in the fluid state detection apparatus 20A. When electromagnetic waves are emitted toward the lubricating oil in the container 10A, this RFID reader 25 reads the information from the RFID tag 12A. When electromagnetic waves are emitted toward the lubricating oil in the container 10B, the RFID reader 25 reads the information from the RFID tag 12B. Furthermore, the state of the lubricating oil detected by the detection unit 23 is associated with the information read by the RFID reader 25 when electromagnetic waves are emitted from the transmission unit 21. Thus, it can be identified whether the state of the lubricating oil detected by the detection unit 23 is the state of the lubricating oil in the container 10A or the container 10B.

Note that, although the fluid state detection system 1A detects the lubricating oil in this embodiment, the present invention is not limited thereto, and the state of any kind of fluid can be detected. For example, the fluid state detection system 1A can also detect the degree of contamination of water, and can also detect the quality of a chemical agent or a beverage.

In this embodiment, the containers 10A and 10B may also be reservoir portions for storing the lubricating oil, rather than the aforementioned pipes or the aforementioned portions near the driving portion. The lubricating oil flows relatively gently in the reservoir portion, compared with the lubricating oil in the aforementioned pipes or the aforementioned portions near the driving portion. Accordingly, bubbles are less likely to be generated in the lubricating oil, and the amount of the lubricating oil is less likely to vary. Thus, the state of the lubricating oil can be detected appropriately.

The first embodiment and the second embodiment have been described so far. According to these embodiments, the state of a fluid can be readily detected.

Note that, although the embodiments of this invention have been described in detail with reference to the drawings, the specific configuration is not limited to these embodiments, and also includes any design that does not depart from the gist of the invention, for example.

Furthermore, the present invention is also applicable to hydraulic equipment, for example.

### Reference Signs List

1, 1A Fluid state detection system
10, 10A, 10B Container
11, 11A, 11B Measurement window
12A, 12B RFID tag
20, 20AFluid state detection apparatus
21 Transmission unit
22 Receiving unit
23 Detection unit
24 Notification unit
25 RFID reader
26 Communication unit
30 Management apparatus

## Claims

1. A fluid state detection system for detecting a state of a fluid in a container, comprising:
a measurement window formed in a wall of the container; and
a fluid state detection apparatus for detecting the state of the fluid,
wherein the fluid state detection apparatus comprises:
a transmission unit for emitting electromagnetic waves via the measurement window toward the fluid;
a receiving unit for receiving reflected waves, transmitted waves, or re-radiated scattered waves of the electromagnetic waves emitted from the transmission unit via the measurement window toward the fluid; and
a detection unit for detecting the state of the fluid based on the electromagnetic waves emitted from the transmission unit and the electromagnetic waves received by the receiving unit.

2. The fluid state detection system according to claim 1,
wherein the container is a reservoir portion in which the fluid is stored.

3. The fluid state detection system according to claim 1,
wherein the measurement window is formed in a side wall of the container.

4. The fluid state detection system according to claim 1, further comprising:
a notification unit for giving a notification of a detection result from the detection unit.

5. The fluid state detection system according to claim 1, further comprising:
an identification unit for identifying the container,
wherein the detection unit associates an identification result obtained by the identification unit when the electromagnetic waves are emitted toward the fluid in the container by the transmission unit, with a result of detecting the state of the fluid.
